# EUROPÄISCHE PATENTANMELDUNG

(11) **EP 1 230 916 A2**
(43) Veröffentlichungstag der Anmeldung: **14.08.2002**
(21) Anmeldenummer: 02010785.0
(22) Anmeldetag: 16.12.1997
(51) Int. Cl.: A61K 9/12

(54) **Wässerige Arzneimittelzubereitungen zur Erzeugung treibgasfreier Aerosole**

(30) Priorität: 20.12.1996 DE 19653969
(62) Teilanmeldung aus: 97952949.2
(71) Anmelder: Boehringer Ingelheim Pharma KG, 55216 Ingelheim am Rhein (DE)
(72) Erfinder: Freund, Bernhard, 55435 Gau-Algesheim (DE); Zierenberg, Bernd, 55411 Bingen am Rhein (DE)

(57) **Zusammenfassung**

Die vorliegende Erfindung betrifft die Verwendung von wässerigen Arzneimittellösungen zur Erzeugung treibgasfreier Aerosole und wässrige Arzneimittelzubereitungen enthaltend Tiotropiumbromid, Ipratropiumbromid und Salbutamol oder 3-[(Hydroxydi-2-thienylacetyl)oxy]-8,8- dimethyl-8-azoniabicyclo[3.2.1]oct-6-en-bromid.

## Beschreibung

Die vorliegende Erfindung betrifft Arzneimittelzubereitungen in Form von wässerigen Lösungen zur Erzeugung treibgasfreier Aerosole für die Inhalation.

Die Anwendung von Dosieraerosolen ist in den letzten 20 Jahren fester Bestandteil bei der Therapie obstruktiver Lungenerkrankungen, insbesondere von Asthma, gewesen. Überlicherweise wurden als Treibgase Fluorchlorkohlenwasserstoffe eingesetzt. Nachdem das ozonschädigende Potential dieser Treibgase erkannt worden war, wurden vermehrt Anstrengungen unternommen, Alternativen hierzu zu entwickein. Als eine Alternative bietet sich die Entwicklung von Verneblern an, bei denen wässerige Lösungen pharmakologisch aktiver Stoffe unter hohem Druck so versprüht werden, daß Nebel inhalierbarer Teilchen entstehen. Der Vorteil dieser Vernebler ist, daß auf den Einsatz von Treibgasen völlig verzichtet werden kann.

Solche Vernebler sind beispielsweise in der PCT-Patentanmeldung WO91/14468 beschrieben, auf die hiermit inhaltlich Bezug genommen wird. Bei den dort beschriebenen Verneblern werden wirkstoffhaltige Lösungen definierter Volumina unter Anwendung hoher Drucke durch kleine Düsen versprüht, so daß inhalierbare Aerosole mit einer mittleren Teilchengröße zwischen 3 und 10 Mikrometer entstehen. Eine weiterentwickelte Ausführungsform der oben genannten Vernebler ist in der PCT/EP96/04351 beschrieben. Der in Figur 6 dargestellte Vernebler trägt das Warenzeichen Respimat®.

Überlicherweise sind die zur Inhalation bestimmten Arzneistoffe in einer wässerigen oder ethanolischen Lösung gelöst, wobei je nach den Lösungseigenschaften der Wirkstoffe auch Lösungsmittelgemische aus Wasser und Ethanol geeignet sind.

Weitere Bestandteile des Lösungsmittels sind neben Wasser und/oder Ethanol gegebenenfalls weitere Cosolventien, ebenfalls kann die Ärzneimittelzubereitung Geschmackstoffe und weitere pharmakologische Hilfsstoffe enthalten.
Beispiele für Cosolventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester, Cosolventien sind dazu geeignet, die Löslichkeit von Hilfsstoffen und gegebenenfalls der Wirkstoffe zu erhöhen.

Der Anteil des gelösten Arzneistoffes an der fertigen Arzneimittelzubereitung beträgt zwischen 0.001 und 30 % - vorzugsweise zwischen 0.05 und 3 %, insbesondere 0.01 bis 2 % (Gewicht/Volumen). Die maximale Konzentration des Arzneistoffes ist abhängig von der Löslichkeit im Lösungsmittel und von der erforderlichen Dosierung zur Erzielung der gewünschten therapeutischen Wirkung.

Als Arzneistoffe in der neuen Zubereitungen können alle Substanzen verwendet werden, die für die inhalative Anwendung geeignet sind und in dem vorgegebenen Lösungsmittel löslich sind. Von besonderem Interesse sind Arzneistoffe zur Behandlung von Atemwegserkrankungen.
Es handelt sich demnach insbesondere um Betamimetica, Anticholinergika, Antiallergika, Antihistaminika und um Steroide sowie Wirkstoffkombinationen davon.

In Reihenuntersuchungen wurde nun gefunden, daß die eingangs beschriebenen Vernebler bei der Verwendung von wässerigen Arzneimittellösungen (überlicherweise wird bidestilliertes oder entmineralisiertes (lonenaustauscher) Wasser als Lösungsmittel eingesetzt. Sprühanomalien aufweisen können. Diese Sprühanomalien stellen sich als eine Veränderung des Sprühbildes des Aerosols dar, mit der Konsequenz, daß im Extremfall aufgrund der veränderten mittleren Tröpfchengrößenverteilung (Veränderung des lungengängigen Anteils des Aerosols) eine exakte Dosierung der zu applizierenden Einzeldosis für den Patienten nicht mehr gewährleistet ist. Diese Sprühanomalien stellen sich besonders dann ein, wenn der Vernebler intervallartig, beispielsweise mit Ruhepausen von ca. 3 und mehr Tagen zwischen den Einzelbetätigungen betrieben wird. Möglicherweise sind diese Sprühanomalien, die im Extremfall bis zu einem Ausfall des Gerätes führen können, auf mikroskopische Ablagerungen im Bereich des Düsenausgangs zurückzuführen.

Überraschenderweise wurde gefunden, daß diese Sprühanomalien nicht mehr auftreten, wenn die zu versprühenden wässerigen Arzneimittelzubereitungen eine definierte wirksame Menge eines Komplexbildners, insbesondere von EDTA (Ethylendiamintetraessigsäure) bzw. deren Salze. Die erfindungsgemäßen wässerigen Arzneimittelzubereitungen enthalten als Lösungsmittel Wasser, gegebenenfalls kann zur Erhöhung der Löslichkeit bis zu 70 % (v/v, bevorzugt zwischen 30 und 60 % (v/v) Ethanol zugesetzt werden.

Weitere pharmakologische Hilfsstoffe wie beispielsweise Konservierungsmittel insbesondere Benzalkoniumchlorid, können zugesetzt sein. Die bevorzugte Menge an Konservierungsstoff, insbesondere an Benzalkoniumchlorid liegt zwischen 8 und 12 mg/100 ml Lösung.

Geeignete Komplexbildner sind solche die pharmakologisch verträglich sind, insbesondere solche die bereits arzneimittelrechtlich zugelassen sind. Besonders geeignet sind EDTA, Nitrilotriessigsäure, Zitronensäure und Ascorbinsäure wie auch deren Salze. Besonders bevorzugt ist das Dinatriumsalz der Ethylendiamtetraessigsäure.

Die Menge an Komplexbildner wird so gewählt, daß eine wirksame Menge an Komplexbildner zugesetzt wird, so daß keine Sprühanomalien mehr auftreten.

Für den Komplexbildner Na-EDTA liegt die wirksame Menge zwischen 10 und 1000 mg / 100 ml Lösung, insbesondere zwischen 10 und 100 mg/ 100ml Lösung. Der bevorzugte Bereich der Menge an Komplexbildner beträgt zwischen 25 und 75 mg/100 ml Lösung, insbesondere zwischen 25 und 50 mg/100 ml Lösung.

Die nachfolgend genannten Verbindungen können prinzipiell als Wirkstoff oder Wirkstoffkombination in der erfindungsgemäßen wässerigen Arzneimittelzubereitung verwendet werden. In Einzelfällen kann es zur Verbesserung der Löslichkeit erforderlich sein, entweder einen höheren Gehalt an Ethanol oder aber einen Lösungsvermittier einzusetzen.

Tiotropiumbromid, 3-[(hydroxydi-2-thienylacetyl)oxy]-8,8-dimethyl-,8-azoniabicyclo[3.2.1]oct-6-en-bromid

| Als Betamimetika: | | | |
|---|---|---|---|
| Bambuterol | Bitolterol | Carbuterol | Formoterol |
| Clenbuterol | Fenoterol | Hexoprenalin | Procaterol |
| Ibuterol | Pirbuterol | Salmeterol | Tulobuterol |
| Reproterol | Salbutamol | Sulfonterol | Terbutalin |

1-(2-Fluor-4-hydroxyphenyl)-2-[4-(1-benzimidazolyl)-2-methyl-2-butylamino]ethanol, erythro-5'-Hydroxy-8'-(1-hydroxy-2-isopropylaminobutyl)-2H-1,4-benzoxazin-3-(4H)-on, 1-(4-Amino-3-chlor-5-trifluormethylphenyl)-2-tert.-butyl-amino)ethanol, 1-(4-Ethoxycarbonylamino-3-cyan-5-fluorphenyl)-2-(tert.-butylamino)ethanol.

Als Anticholinergika:
Ipratropiumbromid
Oxitropiumbromid
Trospiumchlorid
Benzilsäure-N-β-fluorethylnortropinestermethobromid

Als Steroide:
Budesonid
Beclometason (bzw. das 17,21-Dipropionat)
Dexamethason-21-isonicotinat
Flunisolid

Als Antiallergika:
Dinatriumcromoglicat
Nedocromil
Epinastin

Beispiele für Steroide, die als Wirkstoff in der erfindungsgemäßen Arzneimittelzubereitung verwendet werden können sind:

| | |
|---|---|
| Seratrodast | Mycophenolate mofetil |
| Pranlukast | Zileuton |
| Butixocort | Budesonide |
| Deflazacort | |
| Fluticasone | Promedrol |
| Mometasone furoate | Tipredane |
| Beclomethasone, Douglas | Icomethasone enbutate |
| Ciclometasone | Cloprednol |
| Fluocortin butyl | Halometasone |
| Deflazacort | Alclometasone |
| Ciclometasone | Alisactide |
| Prednicarbate | Hydrocortison-butyratpropionat |
| Tixocortol-pivalate | Alclometasone-dipropionate |
| Lotrisone | Canesten-HC |
| Deprodone | Fluticasone-propionate |
| Methylprednisolone-Aceponate | Halopredone-acetate |
| Mometasone | Mometasone-furoate |
| Hydrocortisone-aceponate | Mometasone |
| Ulobetasol-propionate | Aminoglutethimide |
| Triamcinolone | Hydrocortisone |
| Meprednisone | Fluorometholone |
| Dexamethasone | Betamethasone |
| Medrysone | Fluclorolone acetonide |
| Fluocinolone acetonide | Paramethasone-acetate |
| Deprodone Propionate | Aristocort-diacetat |
| Fluocinonide | Mazipredone |
| Difluprednate | Betamethasoe valerate |
| Dexamethasonisonicotinat | Beclomethasone-Dipropionate |
| Fluocortoloncapronat | Formocortal |
| Triamcinolon-Hexacetonide | Cloprednol |
| Formebolone | Clobetason |
| Endrisone | Flunisolide |
| Halcinonide | Fluazacort |
| Clobetasol | Hydrocortison-17-Butyrat |
| Diflorasone | Fluocortin |
| Amcinonide | Betamethasone Dipropionate |
| Cortivazol | Betamethasonadamantoat |
| Fluodexan | Trilostane |
| Budesonide | Clobetasone |
| Demetex | Trimacinolon Benetonid |

9. alpha.-chloro-6.alpha.-fluoro-11.beta.17.alpha.-dihydroxy-16.alpha.-methyl-3-oxo-1,4-androstadiene-17.beta.-carboxylic acid-methylester-17-propionate.

Weitere besonders geeignete Wirkstoffe zur Herstellung von wässerigen Arzneimittelzubereitung für die inhalative Anwendung sind:
β-Sympatico-mimetica;
z. B. Fenoterol, Salbutamol, Formoterol, Terbutalin;
Anticholinergica; z. B. Ipatropium, Oxitropium, Thiotropium; Steroide; z. B. Beclomethason dipropionat, Budesonid, Flunisolid; Peptide;z. B. Insulin; Schmerzmittel; z. B. Fentanyl.

Es versteht sich von selbst, daß falls erforderlich solche pharmakologische verträgliche Salzformen eingesetzt werden, die sich in dem erfindungsgemäßen Lösemittel lösen.

Im Nachfolgenden wird der Vorteil der erfindungsgemäßen Arzneimittelzubereitung durch Beispiele näher erläutert.

Als Arzneistofflösung wurde Ipatropiumbromidlösung (c = 333 mg/100 ml) mit den pH-Wert 3.4 und dem Konservierungsmittel Benzalkoniumchlorid (c = 10 mg/100 ml) verwendet. Die getesteten Lösungen enthielten entweder kein EDTA bzw. EDTA in der Konzentration c = 0.1 mg, 1 mg, 50 mg und 75 mg/100 ml als Dinatriumsalz.

Für den Test wurden jeweils unbenutzte Respimatgeräte (technische Daten: Volumen der applizierten Arzneimittelzubereitung ca. 15 µl, Druck ca. 300 bar, 2 Strahlen impaktiert aus zwei Düsenöffnungen der Größe 5 x 8 µm) .eingesetzt. Der Abhubmodus wurde für den Test so gelegt, daß die Geräte 5mal betätigt, dann 3 Tage ruhten, anschließend wieder 5mal betätigt wurden und in diesen Intervallrhythmus weiter betrieben wurden. In jeder Meßreihe kamen 15 Geräte zur Untersuchung, die Ergebnisse bzgl. Sprühanomalien sind in der Tab. 1 zusammengestellt.

**Tab. 1**

| Versuchs Nr. | Konzentration vonEDTA in mg/100 ml | Anzahl der Geräte mit Sprühanomalien | getesteter Zeitraum in Tagen |
|---|---|---|---|
| 1 | 0 mg/100 ml | 2 | 20 |
| 2 | 0 mg/100 ml | 5 | 9 |
| 3 | 0.1 mg/100 ml | 5 | 6 |
| 4 | 1 mg/100 ml | 6 | 6 |
| 5 | 50 mg/100 ml | 0 | 200 |
| 6 | 50 mg/100 ml | 0 | 200 |
| 7 | 75 mg/100 ml | 0 | 200 |
| 8 | 75 mg/100 ml | 0 | 200 |

| Formulierungsbeispiele (für Fenoterol und Ipatropium bromid) | |
|---|---|
| Bestandteile | Zusammensetzung in mg/100 ml |
| Fenoterol | 833,3 mg |
| Benzalkoniumchlorid | 10,0 mg |
| EDTA* | 50,0 mg |
| HCl (1n) | ad pH 3.2 |
| | |

| Bestandteile | Zusammensetzung in mg/100 ml |
|---|---|
| Ipatropium bromid | 333,3 mg |
| Benzalkoniumchlorid | 10,0 mg |
| EDTA* | 50,0 mg |
| HCl (1n) | ad pH 3.4 |

In Analogie zu den obigen Beispielen wurden folgende Lösungen hergestellt.

| Wirkstoff | Konzentration mg/100 ml | Benzalkoniumchlorid | EDTA* | Lösungsmittel |
|---|---|---|---|---|
| Berotec | 104-1667 | 10 mg | 50 mg | Wasser |
| Atrovent | 83-1333 | 10 mg | 50 mg | Wasser |
| Berodual | | | | |
| (Atrovent) | 42-667 | 10 mg | 50 mg | Wasser |
| (Berotec) | 104-1667 | 10 mg | 50 mg | Wasser |
| Salbutamol | 104-1667 | 10 mg | 50 mg | Wasser |
| Combivent | | | | |
| (Atrovent) | 167-667 | 10 mg | 50 mg | Wasser |
| (Salbutamol) | 833-1667 | 10 mg | 50 mg | Wasser |
| Ba 679 Br (Tiotropiumbromid) | 4-667 | 10 mg | 50 mg | Wasser |
| BEA 2108 Br | 17-833 | 10 mg | 50 mg | Wasser |
| Oxivent | 416-1667 | 10 mg | 50 mg | Wasser |

| | | | | |
|---|---|---|---|---|
| *In Form des Dinatriumsalzes | | | | |

Für die Wirkstoffe sind abhängig von der Dosis pro Hub und ihrer Löslichkeit ein Konzentrationsbereich von 10 mg bis 20 000 mg/100 ml denkbar. Die angegebenen Dosierungen berechnen sich auf der Grundlage einer therapeutisch wirksamen Einzeldosierung von ca. 12 Mikroliter pro Hub. Bei einem geänderten Volumen der Einzeldosierung können sich die Wirkstoffkonzentrationen der Arzneimittelzubereitung ändern.

Für den Komplexbildner (beispielhaft DiNa-EDTA) liegt der Konzentrationsbereich zwischen 10 bis 1000 mg/100 ml (abhängig ebenfalls vom pH-Wert der Lösung). Der bevorzugte Bereich liegt zwischen 25 mg bis 100 mg/100 ml.

Die Menge von Benzalkoniumchlorid sollte im Bereich von 8 bis 12 mg / 100 ml liegen.

Die Lösungen wurden mit 0.1 bzw. 1N HCI auf einen pH von 3.2 bzw. 3.4 eingestellt. Alle Konzentrationsangaben beziehen sich auf 100 ml fertige Wirkstofflösung.

## Patentansprüche

1. Verwendung einer wässerigen Arzneimittelzubereitung in Form einer Lösung zur Erzeugung treibgasfreier Aerosole enthaltend und einen pharmakologisch aktiven Wirkstoff oder eine Wirkstoffkombination ausgewählt aus der Gruppe Fenoterol, Fenoterolhydrobromid, Ipratropiumbromid, Salbutamol, Tiotropiumbromid, 3-[(Hydroxydi-2-thienylacetyl)oxy]-8,8- dimethyl-8-azoniabicyclo[3.2.1]oct-6-en-bromid, Oxitropimbromid, Ipratropiumbromid in Kombination mit Fenoterolhydrobromid, Ipratropiumbromid in Kombination mit Salbutamol, einen Komplexbildner und optional Ethanol in einer Menge von bis zu 70 Vol.%.

2. Verwendung nach Anspruch 1, **dadurch gekennzeichnet, dass** der Komplexbildner Nitrilotriessigsäure, Zitronensäure, Ascorbinsäure oder deren Salze ist.

3. Verwendung nach einem der Ansprüche 1 oder 2, **dadurch gekennzeichnet, dass** der Komplexbildner EDTA oder dessen Salze ist.

4. Verwendung nach einem der Ansprüche 1 bis 3, **dadurch gekennzeichnet, dass** die Konzentration des Komplexbildners zwischen 25 und 100 mg/100 ml Lösung beträgt.

5. Verwendung nach einem der Ansprüche 1 bis 4, **dadurch gekennzeichnet, dass** die Arzneimittelzubereitung bis zu 60 % (v/v) Ethanol enthält.

6. Verwendung nach einem der Ansprüche 1 bis 4, **dadurch gekennzeichnet, dass** die Arzneimittelzubereitung zwischen 30 und 60 % (v/v) Ethanol enthält.

7. Verwendung nach einem der Ansprüche 1 bis 6, **dadurch gekennzeichnet, dass** die Arzneimittelzubereitung den Wirkstoff in einer Konzentration von 0.001 bis 2 g/100 ml Lösung enthält.

8. Arzneimittelzubereitung in Form einer wässrigen Lösung enthaltend Tiotropiumbromid als aktiven Wirkstoff, einen pharmakologisch verträglichen Komplexbildner und optional Ethanol in einer Menge von bis zu 70 Vol.%.

9. Arzneimittelzubereitung nach Anspruch 9 in Form einer Lösung gemäß einem der Ansprüche 2 bis 7.

10. Arzneimittelzubereitung nach einem der Ansprüche 8 oder 9, **dadurch gekennzeichnet, dass** die Arzneimittelzubereitung kein Ethanol enthält.

11. Arzneimittelzubereitung in Form einer wässrigen Lösung enthaltend Ipratropiumbromid und Salbutamol als aktive Wirkstoffkombination, einen pharmakologisch verträglichen Komplexbildner und optional Ethanol in einer Menge von bis zu 70 Vol.%.

12. Arzneimittelzubereitung nach Anspruch 11 in Form einer Lösung gemäß einem der Ansprüche 2 bis 7.

13. Arzneimittelzubereitung nach einem der Ansprüche 11 oder 12, **dadurch gekennzeichnet, dass** die Arzneimittelzubereitung kein Ethanol enthält.

14. Arzneimittelzubereitung in Form einer wässrigen Lösung enthaltend 3-[(Hydroxydi-2-thienylacetyl)oxy]-8,8- dimethyl-8-azoniabicyclo[3.2.1]oct-6-enbromid als aktiven Wirkstoff und einen pharmakologisch verträglichen Komplexbildner und optional Ethanol in einer Menge von bis zu 70 Vol.%.

15. Arzneimittelzubereitung nach Anspruch 13 in Form einer Lösung gemäß einem der Ansprüche 2 bis 7.
